# EUROPEAN PATENT SPECIFICATION

(11) **EP 3 344 052 B1**
(45) Date of publication and mention of the grant of the patent: **23.10.2019**
(21) Application number: 16760689.6
(22) Date of filing: 30.08.2016
(51) Int. Cl.: A23C 9/123, C12R 1/225, A61K 35/747, C12N 1/20, A23K 10/18, A23L 33/135

(54) **LACTOBACILLUS FERMENTUM BACTERIA INHIBITING POST-ACIDIFICATION**
NACHSÄUERUNG HEMMENDE LACTOBACILLUS-FERMENTUM -BAKTERIEN
BACTÉRIES LACTOBACILLUS FERMENTUM INHIBANT LA POST-ACIDIFICATION

(30) Priority: 31.08.2015 EP 15183194
(43) Date of publication of application: 11.07.2018
(73) Proprietor: Chr. Hansen A/S, 2970 Hoersholm (DK)
(72) Inventor: NIELSEN, Cecilie Lykke Marvig, 2700 Broenshoej (DK); HORNBAEK, Tina, 3460 Birkeroed (DK); RASMUSSEN, Pia, 2610 Roedovre (DK); POULSEN, Lone, 4340 Toelloese (DK)
(86) International application number: PCT/EP2016/070398
(87) International publication number: WO 2017/037052

(56) References cited:
- WO-A1-2012/136830
- WO-A1-2013/153070
- WO-A1-2013/153074
- WO-A2-2011/000879
- BILGIN B ET AL: "Assessment of novel human origin Lactobacillus isolates for the manufacture of probiotic yoghurt-like product", MILCHWISSENSCHAFT, VV GMBH VOLKSWIRTSCHAFTLICHER VERLAG. MUNCHEN, DE, vol. 65, no. 1, 1 January 2010 (2010-01-01), pages 65-69, XP008133925, ISSN: 0026-3788

## Description

### FIELD OF THE INVENTION

The present invention relates to *Lactobacillus fermentum* bacteria which inhibit post-acidification, an effect frequently observed in fermented milk products stored at temperatures at and above refrigeration temperature. The invention further provides adjunct cultures comprising the bacteria, method of producing a fermented milk product using the bacteria or the cultures and the fermented milk products thus obtained, including food, feed and pharmaceutical products.

### BACKGROUND OF THE INVENTION

Lactic acid bacteria (LAB) have been used over decades for increasing the shelf life of food products. During fermentation LAB produce lactic acids as well as other organic acids which cause a reduction of pH of the fermented product. Products having an acidic pH do not support further growth of most microorganisms, including pathogenic and spoilage organisms. However, growth of yeasts and molds is not affected by low pH and often causes spoilage of fermented dairy products.

Traditionally, yoghurt is produced by fermentation of milk with a specific yoghurt starter culture consisting of a mixture of two species of lactic acid bacteria (LAB), *Lactobacillus delbrueckii* subsp. *bulgaricus* and *Streptococcus thermophilus.* The main role of the starter in the production of yoghurt are (i) acidification through the conversion of lactose into lactic acid, (ii) creation of viscous texture e.g. by denaturation of proteins and production of exopolysaccharides, and (iii) development of the typical yoghurt flavor (1).

The typical yoghurt flavor is caused by lactic acid, which imparts an acidic and refreshing taste, and a mixture of various carbonyl compounds like acetone, diacetyl, and acetaldehyde, the latter of which is considered the major flavor component (2).

After termination of fermentation the yoghurt is stored until consumed. During this period, the LAB may cause further acidification of the product, in particular when stored above refrigeration temperatures. This phenomenon is called post-acidification and is generally considered to represent an undesired trait observed in most yoghurt types over time. Post-acidification may contribute to defects such as syneresis, increased reduction of viable counts, accumulation of lactic acid in the product and development of an undesired flavor. It is mainly due to the uncontrollable growth of strains of *L. delbrueckii* subsp, *bulgaricus* at low pH values and refrigerated temperatures (4). It can be influenced to a limited extent by applying good manufacturing practice and by using cultures with reduced post-acidification behavior, as e.g. Chr. Hansen Mild 2.0®. Another possibility to circumvent this problem is to pasteurize the yogurt after its manufacture. Although this manipulation would aid the manufacturers considerably in handling and distribution, yogurt pasteurization is not commonly applied because many consumers wish to obtain fermented milk products containing live microorganisms providing an intact lactase activity. Further, mildly-acidifying yogurt-related cultures containing *L. acidophilus* and bifidobacteria offer the advantage of less post-acidification.

At the same time, it has been observed that starter cultures with antifungal effects, i.e. cultures also known as bioprotective bacterial starter cultures (e.g. Holdbac® YM-C plus and FreshQ®4), appear to increase post-acidification in methods of producing fermented milk products, in particular when the fermented products are stored at ambient temperatures.

As a consequence of these findings, there is still a need to improve the control of post-acidification in methods for producing fermented milk products.

WO2011/000879 A2 discloses a fermented milk product prepared with a starter and *L. fermentum* DSM 22584.

### SUMMARY OF THE I NVENTI ON

The present invention provides a bacterium of the species *Lactobacillus fermentum* selected from the group consisting of: (a) the *Lactobacillus fermentum* strain deposited as DSM32086; (b) the *Lactobacillus fermentum* strain deposited as DSM32087; (c) the *Lactobacillus fermentum* strain deposited as DSM32089; (d) the *Lactobacillus fermentum* strain deposited as DSM32091; or (e) a mutant *Lactobacillus* strain obtainable from one the deposited bacteria according to (a) to (d), wherein the mutant is characterized in that it increases the pH of a fermented milk product comprising the mutant *Lactobacillus fermentum* during storage after fermentation in comparison to a milk product fermented with the same starter culture not containing the *Lactobacillus fermentum,* wherein the increase in pH is at least by a value of 0.1, and wherein the increase in pH is determined after storing a product fermented with a starter culture and the *Lactobacillus fermentum* in a concentration of at least 107 cfu/g over 21 days at 25 °C.

The inventors surprisingly found that the *Lactobacillus fermentum* strains of the present invention not only do not contribute to post-acidification, but actually antagonize post-acidification by increasing the pH value of a fermented milk product produced with these strains. As is shown in the Examples, below, the effect is particularly notable in fermentation processes using starter cultures exhibiting significant post-acidification, including a number of commercially available starter cultures and in particular when used together with commercial antifungal bacteria.

In a related embodiment the present invention also provides a bacterium of the species *Lactobacillus fermentum* characterized in that a fermented milk product comprising the mutant *Lactobacillus fermentum* as defined above maintains a pH above 4.0 when stored for at least 14 days at 25 °C, wherein the fermented milk product is obtained by a method comprising incubating a milk with the *Lactobacillus fermentum* in a concentration of at least 10⁷ CFU/g and with a starter culture, fermenting until a pH of 4.6 is reached, shaking the fermented product and cooling.

The bacteria of the present invention may additionally be characterized in reducing the presence of acetaldehyde as produced by other bacteria in the starter culture. For example, certain *Lactobacillus fermentum* strains of the present invention may further be characterized in having the ability to reduce the concentration of acetaldehyde produced by a starter culture during fermentation in a fermented milk product by at least 50%.

In a related embodiment the *Lactobacillus fermentum* strains of the present invention can further be characterized in that the bacterium secretes diacetyl in a range of 0 to 5 ppm.

The present invention therefore provides the bacteria as described above, compositions comprising the same, methods using the bacteria for producing fermented milk products, as well as the products thus obtained.

### DETAILED DESCRIPTION OF THE INVENTION

The present invention provides a bacterium of the species *Lactobacillus fermentum* selected from the group consisting of: (a) the *Lactobacillus fermentum* strain deposited as DSM32086; (b) the *Lactobacillus fermentum* strain deposited as DSM32087; (c) the *Lactobacillus fermentum* strain deposited as DSM32089; (d) the *Lactobacillus fermentum* strain deposited as DSM32091; or (e) a mutant *Lactobacillus* strain obtainable from one the deposited bacteria according to (a) to (d), wherein the mutant is characterized in that it increases the pH of a fermented milk product comprising the mutant *Lactobacillus fermentum* during storage after fermentation in comparison to a milk product fermented with the same starter culture not containing the *Lactobacillus fermentum,* wherein the increase in pH is at least by a value of 0.1, and wherein the increase in pH is determined after storing a product fermented with a starter culture and the *Lactobacillus fermentum* in a concentration of at least 107 cfu/g over 21 days at 25 °C.

The *Lactobacillus fermentum* strains of the present invention are characterized in that they increase the pH (i.e. counteracts the postacidification) of a fermented milk product comprising the *Lactobacillus fermentum* during storage after fermentation in comparison to a milk product fermented with the same starter culture not containing the *Lactobacillus fermentum,* wherein the increase in pH is at least by a value of 0.1 and is determined after storing the fermented product over 21 days at 25°C. For example, the starter culture used for the preparation of the fermented milk product comprises LAB which are able to decrease the pH of a milk product during fermentation to a value of pH 4.6 in 10 hours or less. For example, the assay may be based on mixtures of *Streptococcus thermophilus* and *Lactobacillus delbrueckii* subsp. *bulgaricus.* Respective mixtures are frequently used for the production of yoghurt and known to cause post-acidification.

The mutant bacterium of the species *Lactobacillus fermentum* is further characterized in that a fermented milk product comprising the *Lactobacillus fermentum* maintains a pH above 4.0 when stored for at least 14 days at 25 °C, wherein the fermented milk product is obtained by a method comprising incubating a milk with the *Lactobacillus fermentum* in a concentration of at least 10⁷ CFU/g and with a starter culture, fermenting until a pH of 4.6 is reached, shaking the fermented product and cooling. It should be understood that the feature specifying that the *Lactobacillus fermentum* strains of the present invention can maintain the pH above 4.0 when stored for at least 14 days at 25 °C merely characterizes the assay generally used to determine the effect. It is not necessary or required that the *Lactobacillus fermentum* strains of the present invention, compositions comprising the same, including food or feed products are in fact stored under these conditions. Again, in one aspect the assay can be carried out using a starter culture comprising LAB which are able to decrease the pH of a milk product during fermentation to a value of pH 4.6 in 10 hours or less. For example, the assay may be based on mixtures of *Streptococcus thermophilus* and *Lactobacillus delbrueckii* subsp. *bulgaricus.*

The *Lactobacillus fermentum* strains of the present invention have particular advantages as they reduce the risk of post-acidification and thus improve the storage stability of food products made with these bacteria, in particular the storage stability under conditions above refrigeration temperatures.

As indicated in the Examples, the effect of the *Lactobacillus fermentum* strains of the present invention of increasing the pH of a fermented milk product was observed when the same were added to the milk prior to fermentation in a concentration of least 10⁷ CFU/g. The invention therefore encompasses the *Lactobacillus fermentum* strains of the present invention in a concentration of at least 10⁷ CFU/g, compositions comprising the same and fermented food products comprising the same. Preferred concentration ranges include a concentration of 10⁷ CFU/g to 10¹¹ CFU/g, 10⁷ CFU/g to 10¹⁰ CFU/g and 10⁷ CFU/g to 10⁹ CFU/g.

Common starter cultures, such as commercially available mixtures of *Streptococcus thermophilus* and *Lactobacillus delbrueckii* subsp. *bulgaricus* are known to produce volatile compounds which contribute significantly to the sensory properties of the fermented products. According to one aspect the bacteria of the invention are further characterized in secreting low amounts or essentially no volatile compounds, which affect the sensory properties of the food product. Acetaldehyde, diacetyl and acetoin are known volatile compounds, which affect the sensory properties of the food product.

In a related embodiment the bacteria of the present invention are characterized in reducing the presence of acetaldehyde as produced by other bacteria in the starter culture. For example, certain *Lactobacillus fermentum* strains of the present invention may further be characterized in having the ability to reduce the concentration of acetaldehyde produced by a starter culture during fermentation in a fermented milk product by at least 50%. Different assays are known in the art for determining the concentration of acetaldehyde in a fermented product and can be used for that purpose in accordance with the present invention. The ability to reduce the concentration of acetaldehyde produced by a starter culture during fermentation in a fermented milk product by at least 50% is preferably determined in an assay comprising:
(1) preparing a fermented milk product by:
   (a) incubating a milk with the *Lactobacillus fermentum* in a concentration of at least 10⁷ CFU/g and with a starter culture,
   (b) fermenting until a pH of 4.6 is reached, and;
(2) storing the fermented milk product at 7±1°C for 14 days;
(3) adding 200 µl of 4N H₂SO₄ to 1 g of the fermented milk product and determining the concentration of acetaldehyde by static head space gas chromatography.

Acetaldehyde is a taste component produced by lactic acid bacteria during fermentation. While the component is desirable in certain applications, it would be advantageous to reduce or avoid the presence of acetaldehyde in other applications. *Lactobacillus fermentum* bacteria reducing the concentration of acetaldehyde in a fermented milk product therefore provide advantages in specific applications, for example when preparing sweetened yoghurt. The *Lactobacillus fermentum* strains of the present invention may for example reduce the concentration of acetaldehyde produced by a starter culture during fermentation in a fermented milk product by at least 75%, at least 95% or at least 98%.

Alternatively or additionally, the *Lactobacillus fermentum* strains of the present invention can be characterized in that the bacterium secretes diacetyl in a range of 0 to 5 ppm. Again, different assays are known in the art for determining the concentration of diacetyl in a fermented product and can be used for that purpose in accordance with the present invention. The ability to reduce the concentration of diacetyl produced by a starter culture during fermentation in a fermented milk product by at least 50% is preferably determined in an assay comprising:
(1) preparing a fermented milk product by:
   (a) incubating a milk with the *Lactobacillus fermentum* in a concentration of at least 10⁷ CFU/g and with a starter culture,
   (b) fermenting until a pH of 4.6 is reached, and;
(2) storing the fermented milk product at 7±1°C for 14 days;
(3) adding 200 µl of 4N H₂SO₄ to 1 g of the fermented milk product and determining the concentration of diacetyl by static head space gas chromatography.

*Lactobacillus fermentum* bacteria secreting low concentrations of diacetyl have advantages in methods of producing food products with these bacteria, as the strains that produce high concentrations of these volatile compounds affect the taste of the final product and can therefore not be used for all applications. *Lactobacillus fermentum* strains of the present invention can be characterized in that the bacterium secretes diacetyl in a range of 0 to 3 ppm or 0 to 2ppm.

The following strains are described herein. Only strains belonging to (c), (d), (f) and (h) are according to the invention.
(a) the *Lactobacillus fermentum* strain CHCC12798 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32084;
(b) the *Lactobacillus fermentum* strain CHCC12797 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32085;
(c) the *Lactobacillus fermentum* strain CHCC14591 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32086;
(d) the *Lactobacillus fermentum* strain CHCC14588 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32087;
(e) the *Lactobacillus fermentum* strain CHCC15844 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32088;
(f) the *Lactobacillus fermentum* strain CHCC15865 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32089;
(g) the *Lactobacillus fermentum* strain CHCC15847 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32090;
(h) the *Lactobacillus fermentum* strain CHCC15848 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32091;
(i) the *Lactobacillus fermentum* strain CHCC15926 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 32096;
(j) the *Lactobacillus fermentum* strain CHCC2008 as deposited at German Collection of Microorganisms and Cell Cultures (Deutsche Sammlung von Mikroorganismen und Zellkulturen GmbH; DSMZ), Inhoffenstr. 7B, D-38124 Braunschweig under the accession No.: 22584;
(k) a mutant strain obtainable from one the deposited bacteria according to (a) to (j), wherein the mutant is characterized in that it increases the pH of a fermented milk product comprising the *Lactobacillus fermentum* during storage after fermentation in comparison to a milk product fermented with the same starter culture not containing the *Lactobacillus fermentum.*

The increase in pH caused by the mutant reaches a value of at least 0.1. The increase is determined after storing the fermented product over 21 days at 25 °C.

In the context of the present application, the term "lactic acid bacteria" or "LAB" is used to refer to food-grade bacteria producing lactic acid as the major metabolic end-product of carbohydrate fermentation. These bacteria are related by their common metabolic and physiological characteristics and are usually Gram positive, low-GC, acid tolerant, non-sporulating, non-respiring, rod-shaped bacilli or cocci. During the fermentation stage, the consumption of lactose by these bacteria causes the formation of lactic acid, reducing the pH and leading to the formation of a protein coagulum. These bacteria are thus responsible for the acidification of milk and for the texture of the dairy product. As used herein, the term "lactic acid bacteria" encompasses, but is not limited to, bacteria belonging to the genus of *Lactobacillus* spp., *Bifidobacterium* spp., *Streptococcus* spp., *Lactococcus* spp., such as *Lactobacillus delbrueckii subsp. bulgaricus, Streptococcus thermophilus, Lactobacillus lactis, Bifidobacterium animalis, Lactococcus lactis, Lactobacillus paracasei, Lactobacillus plantarum, Lactobacillus helveticus*, *Lactobacillus acidophilus, Bifidobacterium breve* and *Leuconostoc* spp.

Depending on the optimum temperature for propagation, LAB are characterized as mesophilic or thermophilic LAB. The term "mesophile" refers to microorganisms that thrive best at moderate temperatures. The term "mesophilic fermentation" herein refers to fermentation at a temperature between about 22°C and about 35°C. The term "mesophilic fermented milk product" refers to fermented milk products prepared by mesophilic fermentation of a mesophilic starter culture and include such fermented milk products as buttermilk, sour milk, cultured milk, smetana, sour cream and fresh cheese, such as quark, tvarog and cream cheese. The industrially most useful mesophilic bacteria include *Lactococcus* spp. and *Leuconostoc* spp.

The term "thermophile" refers to microorganisms that thrive best at high temperatures. The term "thermophilic fermentation" refers to fermentation methods carried out at a temperature between about 35°C and about 45°C. The term "thermophilic fermented milk product" refers to fermented milk products prepared by thermophilic fermentation using a thermophilic starter culture and include such fermented milk products as set-yoghurt, stirred-yoghurt, strained yoghurt and drinking yoghurt. The industrially most useful thermophilic bacteria include *Streptococcus* spp. and *Lactobacillus* spp.

As will be outlined below, the present invention encompasses methods using mesophilic and thermophilic fermentation.

The terms "inhibit" in relation to fungi, yeasts and molds refers to a decrease in the growth or sporulation or a reduction in the number or in the concentration of fungi, yeasts and molds, for example in food products and/or on the surface of food products comprising the bacteria of the present invention in relation to food products which do not comprise such bacteria. The extent of inhibition provided by the *Lactobacillus fermentum* bacteria of the present invention is preferably determined by growth on agar solidified fermented milk in the presence and absence of the *Lactobacillus fermentum* bacteria.

In the present context, the term "mutant" should be understood as a strain derived from a strain of the invention by means of e.g. genetic engineering, radiation and/or chemical treatment. It is preferred that the mutant is a functionally equivalent mutant, e.g. a mutant that has substantially the same, or improved, properties in particular in relation to the effects on inhibiting post-acidification, as the deposited strain. Such a mutant is a part of the present invention. Especially, the term "mutant" refers to a strain obtained by subjecting a strain of the invention to any conventionally used mutagenization treatment including treatment with a chemical mutagen such as ethane methane sulphonate (EMS) or N-methyl-N'-nitro-N-nitroguanidine (NTG), UV light or to a spontaneously occurring mutant. A mutant may have been subjected to several mutagenization treatments (a single treatment should be understood one mutagenization step followed by a screening/selection step), but it is presently preferred that no more than 20, or no more than 10, or no more than 5, treatments (or screening/selection steps) are carried out. In a presently preferred mutant, less than 5%, or less than 1% or even less than 0.1% of the nucleotides in the bacterial genome have been shifted with another nucleotide, or deleted, compared to the mother strain.

The use of the terms "a" and "an" and "the" and similar referents in the context of describing the invention (especially in the context of the following claims) are to be construed to cover both the singular and the plural, unless otherwise indicated herein or clearly contradicted by context.

The present invention further provides compositions comprising at least one bacterium of the species *Lactobacillus fermentum* characterized in that a fermented milk product comprising the *Lactobacillus fermentum* maintains a pH above 4.0 when stored for at least 14 days at 25 °C in the assay as described above.

Respective compositions may comprise numerous further bacteria including LABs. A preferred composition of the present invention is therefore characterized in that the composition further comprises at least one further bacterium selected from one or more of the following genera and species *Lactobacillus* spp., *Bifidobacterium* spp., *Streptococcus* spp., *Lactococcus* spp., such as *Lactobacillus delbrueckii subsp. bulgaricus, Streptococcus thermophilus, Lactobacillus lactis, Bifidobacterium animalis, Lactococcus lactis, Lactobacillus paracasei, Lactobacillus plantarum, Lactobacillus helveticus*, *Lactobacillus acidophilus, Bifidobacterium breve* and *Leuconostoc* spp.

In a particularly preferred embodiment, the compositions of the present invention comprise at least one bacterium of the species *Lactobacillus fermentum* inhibiting post-acidification as described above and one or more second bacterium. In one embodiment, several different strains of the *Lactobacillus fermentum* bacteria of the present invention are combined. The further bacteria can for example be selected from bacteria with antifungal activities:
(a) *Lactobacillus rhamnosus* bacterium of strain CHCC15860 as deposited with the German Collection of Microorganisms and Cell Cultures (DSMZ) under accession No. DSM32092;
(b) *Lactobacillus rhamnosus* bacterium of strain CHCC5366 as deposited with the German Collection of Microorganisms and Cell Cultures (DSMZ) under accession No. DSM23035;
(c) *Lactobacillus rhamnosus* bacterium of strain CHCC12697 as deposited with the German Collection of Microorganisms and Cell Cultures (DSMZ) under accession No. DSM24616;
(d) *Lactobacillus paracasei* bacterium of strain CHCC12777 as deposited with the German Collection of Microorganisms and Cell Cultures (DSMZ) under accession No. DSM24651; and
(e) *Lactobacillus paracasei* bacterium of strain CHCC14676 as deposited with the German Collection of Microorganisms and Cell Cultures (DSMZ) under accession No. DSM25612.

The compositions of the present invention may in addition comprise numerous further components, including one or more cryoprotective compounds as well as flavoring compounds.

LAB are most commonly added in the form of a starter culture to milk. The term "starter" or "starter culture" as used in the present context refers to a culture of one or more food-grade micro-organisms, in particular lactic acid bacteria, which are responsible for the acidification of the milk base. Starter cultures may be fresh, but are most frequently frozen or freeze-dried. These products are also known as "Direct Vat Set" (DVS) cultures and are produced for direct inoculation of a fermentation vessel or vat for the production of a dairy product, such as a fermented milk product or a cheese. Respective starter cultures are commercially available from numerous sources and include F-DVS YoFlex Mild 2.0, F-DVS YF-L901, FD-DVS YF-812 and F-DVS CH-1, four cultures commercially available from Chr. Hansen containing mixtures of *Streptococcus thermophilus* and *Lactobacillus delbrueckii* subsp. *bulgaricus.*

In one aspect the present invention therefore provides compositions in the form of a solid frozen or freeze-dried starter culture comprising lactic acid bacteria in a concentration of at least 10⁹ colony forming units per g of frozen material or in a concentration of at least 10¹⁰ colony forming units per g of frozen material or in a concentration of at least 10¹¹ colony forming units per g of frozen material which compositions include a bacterium of the species *Lactobacillus fermentum* which inhibits post-acidification as described above.

There is also described a bacterium of the species *Lactobacillus fermentum,* wherein the bacterium is characterized in that it increases the pH of a fermented milk product comprising the *Lactobacillus fermentum* during storage after fermentation in comparison to a milk product fermented with the same starter culture not containing the *Lactobacillus fermentum,*
wherein the increase in pH is at least by a value of 0.1,
and wherein the increase in pH is determined after storing a product fermented with a starter culture and the *Lactobacillus fermentum* in a concentration of at least 10⁷ cfu/g over 21 days at 25 °C, and
wherein the bacterium has the ability to reduce the concentration of acetaldehyde produced by a starter culture during fermentation in a fermented milk product by at least 50%.

In a further embodiment the present invention provides methods of producing a fermented milk product which comprise adding the *Lactobacillus fermentum* bacterium which inhibits post-acidification as described above or the composition comprising the same to milk or to a milk product and fermenting the mixture at a temperature between about 22°C and about 43°C until a pH of less than 4.6 is reached.

In the context of the present application, the term "milk" is broadly used in its common meaning to refer to liquids produced by the mammary glands of animals or by plants. In accordance with the present invention the milk may have been processed and the term "milk" includes whole milk, skim milk, fat-free milk, low fat milk, full fat milk, lactose-reduced milk, or concentrated milk. Fat-free milk is non-fat or skim milk product. Low-fat milk is typically defined as milk that contains from about 1% to about 2% fat. Full fat milk often contains 2% fat or more. The term "milk" is intended to encompass milks from different mammals and plant sources. Mammal sources of milk include, but are not limited to cow, sheep, goat, buffalo, camel, llama, mare and deer. Plant sources of milk include, but are not limited to, milk extracted from soy bean, pea, peanut, barley, rice, oat, quinoa, almond, cashew, coconut, hazelnut, hemp, sesame seed and sunflower seed. In the methods and products of the present invention, milk derived from cows is most preferably used as a starting material for the fermentation.

The term "milk" also includes fat-reduced and/or lactose-reduced milk products. Respective products can be prepared using methods well known in the art and are commercially available (for example from Select Milk Producers Inc., Texas, USA). Lactose-reduced milk can be produced according to any method known in the art, including hydrolyzing the lactose by lactase enzyme to glucose and galactose, or by nanofiltration, electrodialysis, ion exchange chromatography and centrifugation.

The term "milk product" or "milk base" is broadly used in the present application to refer to a composition based on milk or milk components which can be used as a medium for growth and fermentation of LAB. The milk product or base comprises components derived from milk and any other component that can be used for the purpose of growing or fermenting LAB.

The fermentation step of the process for manufacturing fermented dairy products comprises the addition of LAB to milk. Fermentation processes used in production of dairy products are well known and a person of ordinary skill can select fermentation process conditions, including temperature, oxygen, amount and characteristics of microorganism(s) and fermentation time.

Prior to fermentation, the milk substrate may be homogenized and pasteurized according to methods known in the art. "Homogenizing" as used herein means intensive mixing to obtain a soluble suspension or emulsion. If homogenization is performed prior to fermentation, it may be performed so as to break up the milk fat into smaller sizes so that it no longer separates from the milk. This may be accomplished by forcing the milk at high pressure through small orifices. "Pasteurizing" as used herein means treatment of the milk substrate to reduce or eliminate the presence of live organisms, such as microorganisms. Preferably, pasteurization is attained by maintaining a specified temperature for a specified period of time. The specified temperature is usually attained by heating. The temperature and duration may be selected in order to kill or inactivate certain bacteria, such as harmful bacteria. A rapid cooling step may follow.

In a particularly advantageous method of the present invention the *Lactobacillus fermentum* bacterium which inhibits post-acidification as described above or the composition comprising the same is added to milk or to a milk product and the mixture is fermented in such a manner that
(a) the concentration of the *Lactobacillus fermentum* bacteria which inhibits post-acidification is at least 1x10⁶ cfu/g or at least 1x10⁷ cfu/g at the termination of fermentation in the fermented milk product; and/or
(b) such that the concentration of the *Lactobacillus fermentum* bacteria which inhibits post-acidification is at least 1x10⁵ cfu/cm² on the surface of the fermented milk product.

This way of proceeding has the advantage that post-acidification inhibiting effect of the *Lactobacillus fermentum* bacterium can be fully used.

The present invention further provides methods of, wherein the fermented product is stored at a temperature above 7°C, preferably at a temperature between 7°C and 25°C. The product may be stored at any time, but is preferably stored for a period of at least 14 days and wherein the pH of the fermented milk product is maintained above pH 4.0 during storage.

The invention further provides methods of producing a food, feed or pharmaceutical product comprising a method of producing a fermented milk product as described above and the food, feed or pharmaceutical product obtainable by this method.

Fermentation is carried out to produce food products, feed products or pharmaceuticals. The terms "fermented milk product", "food" or "feed" product refer to products obtainable by the fermentation methods of the present invention and include cheese, yoghurt, fruit yoghurt, yoghurt beverage, strained yoghurt (Greek yoghurt, Labneh), quark, fromage frais and cream cheese. The term food further encompasses other fermented food products, including fermented meat, such as fermented sausages, and fermented fish products.

The term "cheese" is understood to encompass any cheese, including hard, semi-hard and soft cheeses, such as cheeses of the following types: Cottage, Feta, Cheddar, Parmesan, Mozzarella, Emmentaler, Danbo, Gouda, Edam, Feta-type, blue cheeses, brine cheeses, Camembert and Brie. The person skilled in the art knows how to convert the coagulum into cheese, methods can be found in the literature, see e.g. Kosikowski, F. V., and V. V. Mistry, "Cheese and Fermented Milk Foods", 1997, 3rd Ed. F. V. Kosikowski, L. L. C. Westport, CT. As used herein, a cheese which has a NaCl concentration below 1.7% (w/w) is referred to as a "low-salt cheese".

In the context of the present application, the term "yoghurt" refers to products comprising *Streptococcus thermophilus* and *Lactobacillus delbrueckii subsp. bulgaricus* and optionally other microorganisms such as *Lactobacillus delbrueckii subsp. lactis, Bifidobacterium animalis subsp. lactis, Lactococcus lactis, Lactobacillus acidophilus* and *Lactobacillus paracasei,* or any microorganism derived therefrom. The lactic acid strains other than *Streptococcus thermophilus* and *Lactobacillus delbrueckii subsp. bulgaricus,* are included to give the finished product various properties, such as the property of promoting the equilibrium of the flora. As used herein, the term "yoghurt" encompasses set yoghurt, stirred yoghurt, drinking yoghurt, Petit Suisse, heat treated yoghurt, strained or Greek style yoghurt characterized by a high protein level and yoghurt-like products.

In particular, term "yoghurt" encompasses, but is not limited to, yoghurt as defined according to French and European regulations, e.g. coagulated dairy products obtained by lactic acid fermentation by means of specific thermophilic lactic acid bacteria only (i.e. *Lactobacillus delbrueckii subsp. bulgaricus* and *Streptococcus thermophilus*) which are cultured simultaneously and are found to be live in the final product in an amount of at least 10 million CFU (colony-forming unit) / g. Yoghurts may optionally contain added dairy raw materials (e.g. cream) or other ingredients such as sugar or sweetening agents, one or more flavoring(s), fruit, cereals, or nutritional substances, especially vitamins, minerals and fibers, as well as stabilizers and thickeners. Optionally the yoghurt meets the specifications for fermented milks and yoghurts of the AFNOR NF 04-600 standard and/or the codex StanA-IIa-1975 standard. In order to satisfy the AFNOR NF 04-600 standard, the product must not have been heated after fermentation and the dairy raw materials must represent a minimum of 70% (m/m) of the finished product.

There is also described a food, feed or pharmaceutical product comprising one or more bacteria of the species *Lactobacillus fermentum* which inhibits post-acidification as described above and one or more of:
(a) least one further bacterium selected from one or more of the following genera *Lactococcus* spp., *Streptococcus* spp., *Lactobacillus* spp., *Leuconostoc* spp., *Pseudoleuconostoc* spp., *Pediococcus* spp., *Brevibacterium* spp. and *Enterococcus* spp.;
(b) *Lactobacillus rhamnosus* bacterium of strain CHCC15860 as deposited with the German Collection of Microorganisms and Cell Cultures (DSMZ) under accession No. DSM32092;
(c) *Lactobacillus rhamnosus* bacterium of strain CHCC5366 as deposited with the German Collection of Microorganisms and Cell Cultures (DSMZ) under accession No. DSM23035;
(d) *Lactobacillus rhamnosus* bacterium of strain CHCC12697 as deposited with the German Collection of Microorganisms and Cell Cultures (DSMZ) under accession No. DSM24616;
(e) *Lactobacillus paracasei* bacterium of strain CHCC12777 as deposited with the German Collection of Microorganisms and Cell Cultures (DSMZ) under accession No. DSM24651; and
(f) *Lactobacillus paracasei* bacterium of strain CHCC14676 as deposited with the German Collection of Microorganisms and Cell Cultures (DSMZ) under accession No. DSM25612.

### DESCRI PTI ON OF THE FIGURES

**Figure 1** **:** pH development in fermented milk products over time when stored at (A) 7±1°C or (B) 25±1°C for 21 days. The products are fermented with starter culture only (Reference, Δ) or starter culture in combination with FreshQ®4 (◊), Holdbac® YM-C Plus (o) or Lb. fermentum CHCC14591 (□).
**Figure 2****:** pH development in fermented milk products over time when stored at (A) 7±1°C and (B) 25±1°C for 28 days. The products are fermented with starter culture only, Reference, or starter culture in combination with FreshQ®4, Holdbac®® YM-C Plus or Lb. fermentum strains.
**Figure 3****:** Acetaldehyde levels after storage at 7±1°C for 14 days in fermented milk products fermented with starter culture alone (Reference), or starter cultures in combination with Lb. fermentum strains. LOD: Limit of detection. LOQ: Limit of quantification.
**Figure 4****:** Acetaldehyde levels after storage at 7±1°C for 14 days in fermented milk products fermented with starter culture alone (Reference), or starter cultures in combination with Lb. fermentum CHCC14591. LOD: Limit of detection. LOQ: Limit of quantification.
**Figure 5****:** Acidification curves of four commercial starter cultures, FD-DVS YF-L812, F-DVS YF-L901, F-DVS YoFlex Mild 2.0 and F-DVS CH-1, grown in milk (1% fat and 4.5% protein) at 43°C.
**Figure 6****:** Post-acidification curves of yoghurt fermented with one of four commercial starter cultures, FD-DVS YF-L812, F-DVS YF-L901, F-DVS YoFlex Mild 2.0 and F-DVS CH-1 after storage at 6°C for up to 43 days.
**Figure 7****:** Acetaldehyde levels after storage at 7±1°C for 14 days in fermented milk products fermented with starter culture, FD DVS YF-L812 or F-DVS CH-1, alone (Reference), or starter cultures in combination one of the nine Lb. fermentum strains. LOD: Limit of detection. LOQ: Limit of quantification.

### Example 1:

### Analysis of the effect of Lb. fermentum CHCC14591 on post-acidification

Reduced-fat (1.5% w/v) homogenized milk was heat-treated at 90±1°C for 20 min and cooled immediately. A commercial starter culture (F-DVS Mild 2.0) was inoculated at 0.02% (v/w), and the inoculated milk was distributed into 200 ml bottles. One bottle was inoculated with *Lb. fermentum* CHCC14591 in total concentration of 2 x 10⁷ CFU/g, two bottles were inoculated with two commercial bioprotective cultures (FreshQ®4 and Holdbac® YM-C Plus) in recommended dosages (100U/T and 20 DCU/100L for FreshQ®4 and Holdbac® YM-C Plus, respectively), and one bottle was used as a reference and only inoculated with the starter culture. All bottles were incubated in a water bath at 43±1°C and fermented at these conditions until pH of 4.60±0.1 was reached. After fermentation, the bottles were vigorously shaken to break the coagulum and cooled on ice.

To monitor the effect on post acidification, the four fermented milk samples (starter-only, FreshQ®4, Holdbac® YM-C Plus and *Lb. fermentum* CHCC14591) were stored at 7±1°C and 25±1°C for 21 days and pH was measured on day 1, 7, 14 and 21.

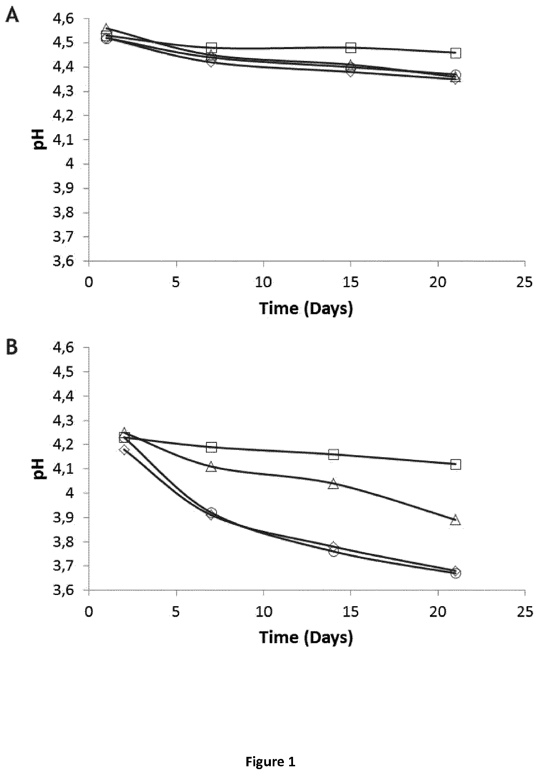

The effect is illustrated in Figure 1, showing that addition of *Lb. fermentum* CHCC14591 during milk fermentation result in lower post acidification compared to the use of the starter culture alone and particularly compared to the use of the two commercial bioprotective cultures both contributing to post-acidification.

### Example 2:

### Analysis of the effect of ten Lb. fermentum strains on post-acidification

Reduced-fat (1.5% w/v) homogenized milk was heat-treated at 90±1°C for 20 min and cooled immediately. A commercial starter culture (F-DVS YF-L901) was inoculated at 0.02% (v/w), and the inoculated milk was distributed into 200 ml bottles. Ten bottles were inoculated with the *Lb. fermentum* strains in concentrations of 1 x 10⁷ CFU/g and one bottle was used as a reference and only inoculated with the starter culture. All bottles were incubated in a water bath at 43±1°C and fermented at these conditions until pH of 4.60±0.1 was reached. After fermentation, the bottles were vigorously shaken to break the coagulum and cooled on ice.

The tested *Lb. fermentum* strains were: *Lb. fermentum* CHCC12798, *Lb. fermentum* CHCC12797, *Lb. fermentum* CHCC14591, *Lb. fermentum* CHCC14588, *Lb. fermentum* CHCC15844, *Lb. fermentum* CHCC15865, *Lb. fermentum* CHCC15847, *Lb. fermentum* CHCC15848, *Lb. fermentum* CHCC15926, and *Lb. fermentum* CHCC2008.

To monitor the effect on post acidification, the eleven fermented milk samples (starter culture alone and starter culture in combination with the ten *Lb. fermentum* strains) were stored at 7±1°C and 25±1°C for 28 days and pH was measured on day 1, 7, 14, 21 and 28.

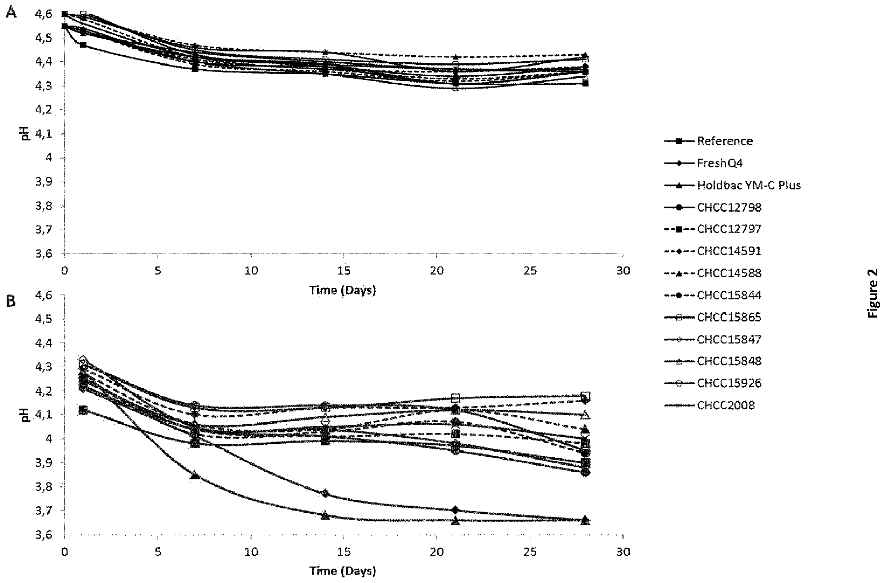

The effects on post-acidification are illustrated in Figure 2 and show that each of the *Lb. fermentum* strains *Lb. fermentum* CHCC12798, *Lb. fermentum* CHCC12797, *Lb. fermentum* CHCC14591, *Lb. fermentum* CHCC14588, *Lb. fermentum* CHCC15844, *Lb. fermentum* CHCC15865, *Lb. fermentum* CHCC15847, *Lb. fermentum* CHCC15848, *Lb. fermentum* CHCC15926, and *Lb. fermentum* CHCC2008 does not contribute to post-acidification or even reduce post-acidification compared to reference yoghurt.

These findings were unexpected and are highly significant, as prior art antifungal food-grade bacteria were observed to increase the post-acidification effects caused by the starter culture.

### Example 3:

### Effect of the ten Lb. fermentum strains on acetaldehyde content

Ten *Lb. fermentum* strains were tested for their ability to lower acetaldehyde content.

Reduced-fat (1.5% w/v) homogenized milk was heat-treated at 90±1°C for 20 min and cooled immediately. A commercial starter culture (F-DVS YF-L901 Yo-Flex®) was inoculated at 0.02% (v/w), and the inoculated milk was distributed into 200 ml bottles. Ten bottles were inoculated with the *Lb. fermentum* strains in concentrations of 1 x 10⁷ CFU/g and one bottle was used as a reference and only inoculated with the starter culture. All bottles were incubated in a water bath at 43±1°C and fermented at these conditions until pH of 4.60±0.1 was reached. After fermentation, the bottles were vigorously shaken to break the coagulum and cooled on ice. The bottles were stored at 7±1°C for 14 days.

On day 14 samples were analyzed for acetaldehyde by static head space gas chromatography (HSGC), a sensitive method for analyzing volatiles in complex matrices. The setup consisted of a Static Head Space sampler connected to Gas Chromatograph with Flame Ionization Detector (FID). For that purpose the following equipment was used:
HS-autosampler: HS40XI, TurboMatrix 110, Perkin Elmer.
HS-software: HSControl v.2.00, Perkin Elmer.
GC: Autosystem XL, Perkin Elmer.
GC-software: Turbochrom navigator, Perkin Elmer.
Column: HP-FFAP 25 m x 0.20 mm x 0.33 µm, Agilent Technologies

Standards of known concentration were used to determine response factors (calibration), controls were used to control that the used response factors were stable within an analytical series as well as in-between series and over time (months). Concentration of volatiles (ppm) in samples and controls was determined using response factors coming from standards. Samples were prepared by adding 200 µl of 4N H₂SO₄ to 1 g yoghurt sample and immediately analyzed by HSGC.

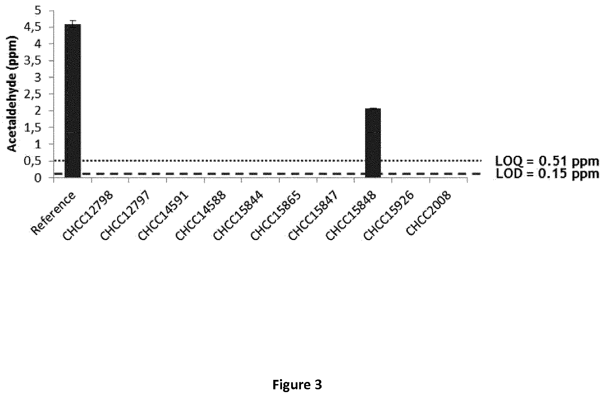

The results are illustrated in Figure 3 and show that each of the strains *Lb. fermentum* CHCC12798, *Lb. fermentum* CHCC12797, *Lb. fermentum* CHCC14591, *Lb. fermentum* CHCC14588, *Lb. fermentum* CHCC15844, *Lb. fermentum* CHCC15865, *Lb. fermentum* CHCC15847, *Lb. fermentum* CHCC15848, *Lb. fermentum* CHCC15926, and *Lb. fermentum* CHCC2008 has the ability to reduce the concentration of acetaldehyde produced by a starter culture during fermentation in a fermented milk product.

### Example 4:

### Effect of one Lb. fermentum strain on acetaldehyde content

One *Lb. fermentum* strain was tested for the ability to lower acetaldehyde content.

Reduced-fat (1.5% w/v) homogenized milk was heat-treated at 90±1°C for 20 min and cooled immediately. A commercial starter culture (F-DVS Mild 2.0) was inoculated at 0.02% (v/w), and the inoculated milk was distributed into two 200 ml bottles. One bottle was inoculated with the *Lb. fermentum* strains in concentrations of 1 x 10⁷ CFU/g and one bottle was used as a reference and only inoculated with the starter culture. Both bottles were incubated in a water bath at 43±1°C and fermented at these conditions until pH of 4.60±0.1 was reached. After fermentation, the bottles were vigorously shaken to break the coagulum and cooled on ice. The bottles were stored at 7±1°C for 14 days.

On day 14 samples were analyzed for acetaldehyde by static head space gas chromatography (HSGC), a sensitive method for analyzing volatiles in complex matrices. The setup consisted of a Static Head Space sampler connected to Gas Chromatograph with Flame Ionization Detector (FID). For that purpose the following equipment was used:
HS-autosampler: HS40XI, TurboMatrix 110, Perkin Elmer.
HS-software: HSControl v.2.00, Perkin Elmer.
GC: Autosystem XL, Perkin Elmer.
GC-software: Turbochrom navigator, Perkin Elmer.
Column: HP-FFAP 25 m x 0.20 mm x 0.33 µm, Agilent Technologies

Standards of known concentration were used to determine response factors (calibration), controls were used to control that the used response factors were stable within an analytical series as well as in-between series and over time (months). Concentration of volatiles (ppm) in samples and controls was determined using response factors coming from standards. Samples were prepared by adding 200 µl of 4N H₂SO₄ to 1 g yoghurt sample and immediately analyzed by HSGC.

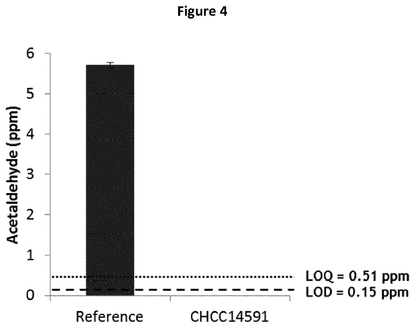

The results are illustrated in Figure 4 and show that *Lb. fermentum* CHCC14591 has the ability to reduce the concentration of acetaldehyde produced by a starter culture during fermentation in a fermented milk product.

### Example 5:

### Functional analysis of commercial starter starter cultures

The three commercial starter cultures included herein were chosen based on their different acidification profiles. Three were frozen, F-DVS CH-1, F-DVS YoFlex Mild 2.0 and F-DVS YF-L901, and one was freeze dried, FD-DVS YF-L812. To test the difference in acidification profiles, semi fat milk was standardized to 1% fat and 4.5% protein with skim milk powder and heat-treated at 85±1°C for 30 min and cooled immediately. One of four different commercial starter cultures (F-DVS CH-1, F-DVS YoFlex Mild 2.0, F-DVS YF-L901 or FD-DVS YF-L812) was inoculated at 0.02% (v/w), and the inoculated milk was distributed into 200 ml bottles. The bottles were incubated in a water bath at 43±1°C and fermented under these conditions until pH 4.5 was reached. The pH was measured continually throughout the fermentation. Subsequently, the bottles were stored at 6°C for 43 for days and pH was measured with intervals of 7 days to determine the level of post-acidification.

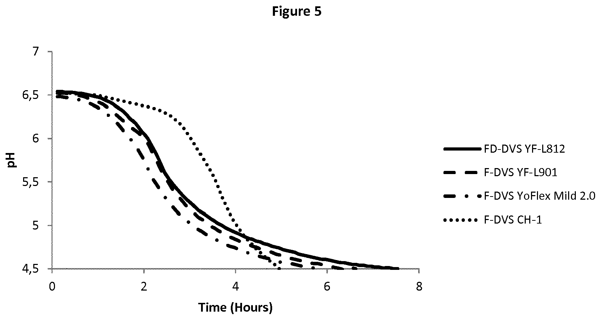

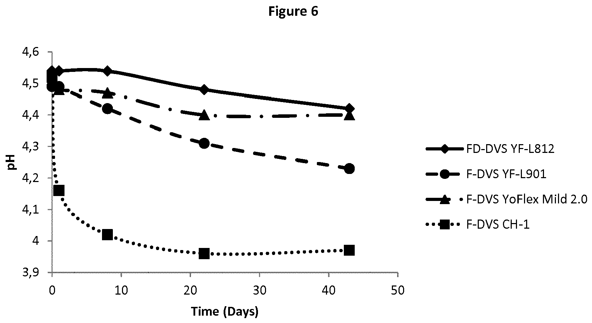

The acidification profiles of the three commercial starter cultures, F-DVS CH-1, F-DVS YoFlex Mild 2.0, F-DVS YF-L901 and FD-DVS YF-L812, are shown in Figure 5. F-DVS CH-1 showed fast fermentation time reaching pH 4.55 in 4.87 hours. F-DVS YoFlex Mild 2.0 showed intermediate fermentation time reaching pH 4.55 in 5.29 hours. FD-DVS YF-L812 and F-DVS YF-L901 showed slower fermentation reaching pH 4.55 in 6.45 and 5.87 hours, respectively. Post-acidification profiles showed very low levels of post-acidification for FD-DVS YF-L812 and F-DVS YoFlex Mild 2.0 (ΔpH=0.12 and ΔpH=0.11 after storage at 6°C for 43 days, respectively), intermediate levels of post-acidification for F-DVS YF-L901 (ΔpH=0.26 after storage at 6°C for 43 days and high degree of post-acidification for F-DVS CH-1 (ΔpH=0.55 after storage at 6°C for 43 days) (Figure 6).

### Example 6:

### Effect of the nine Lb. fermentum strains on acetaldehyde content when fermented with two different starter cultures

Nine *Lb. fermentum* strains were tested for their ability to lower acetaldehyde content.

Reduced-fat (1.5% w/v) homogenized milk was heat-treated at 90±1°C for 20 min and cooled immediately. Milk was inoculated with one of two commercial starter cultures (F-DVS CH-1 or FD-DVS YF-L812) at 0.02% (v/w), and the inoculated milk was distributed into 200 ml bottles. Nine bottles were inoculated with the *Lb. fermentum* strains in concentrations of 1 x 10⁷ CFU/g and one bottle inoculated with each starter culture was used as a reference and only inoculated with the starter culture. All bottles were incubated in a water bath at 43±1°C and fermented at these conditions until pH of 4.55±0.1 was reached. After fermentation, the bottles were vigorously shaken to break the coagulum and cooled on ice. The bottles were stored at 7±1°C for 14 days.

The tested *Lb. fermentum* strains were: *Lb. fermentum* CHCC12798, *Lb. fermentum* CHCC12797, *Lb. fermentum* CHCC14591, *Lb. fermentum* CHCC14588, *Lb. fermentum* CHCC15844, *Lb. fermentum* CHCC15865, *Lb. fermentum* CHCC15847, *Lb. fermentum* CHCC15926, and *Lb. fermentum* CHCC2008.

On day 14 samples were analyzed for acetaldehyde by static head space gas chromatography (HSGC), a sensitive method for analyzing volatiles in complex matrices. The setup consisted of a Static Head Space sampler connected to Gas Chromatograph with Flame Ionization Detector (FID). For that purpose the following equipment was used:
HS-autosampler: HS40XI, TurboMatrix 110, Perkin Elmer.
HS-software: HSControl v.2.00, Perkin Elmer.
GC: Autosystem XL, Perkin Elmer.
GC-software: Turbochrom navigator, Perkin Elmer.
Column: HP-FFAP 25 m x 0.20 mm x 0.33 µm, Agilent Technologies

Standards of known concentration were used to determine response factors (calibration), controls were used to control that the used response factors were stable within an analytical series as well as in-between series and over time (months). Concentration of volatiles (ppm) in samples and controls was determined using response factors coming from standards. Samples were prepared by adding 200 µl of 4N H₂SO₄ to 1 g yoghurt sample and immediately analyzed by HSGC.

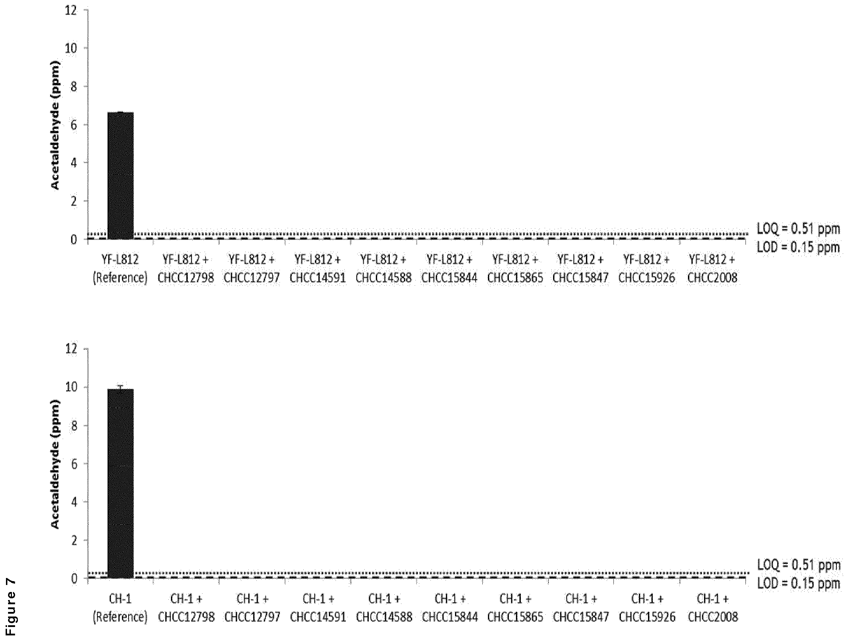

The results are illustrated in Figure 7 and show that each of the strains *Lb. fermentum* CHCC12798, *Lb. fermentum* CHCC12797, *Lb. fermentum* CHCC14591, *Lb. fermentum* CHCC14588, *Lb. fermentum* CHCC15844, *Lb. fermentum* CHCC15865, *Lb. fermentum* CHCC15847, *Lb. fermentum* CHCC15926, and *Lb. fermentum* CHCC2008 has the ability to reduce the concentration of acetaldehyde produced by a starter culture during fermentation in a fermented milk product.

### REFERENCES

**1.** Tamime, A. Y., and H. C. Deeth. 1980. Yoghurt: technology and biochemistry. J. Food Prot. 43:939-977.
2. A. C. S. D. Chaves, M. Fernandez, A. L. S. Lerayer, I. Mierau, M. Kleerebezem, and J. Hugenholtz, 2002, Metabolic Engineering of Acetaldehyde Production by Streptococcus thermophilus
3. Lees, G. J., and G. R. Jago. 1976. Formation of acetaldehyde from threonine by lactic acid bacteria. J. Dairy Res. 43:75-83.
4. Kurmann, J.A. (1982) Die Obersauerung der Joghurtgallerte, ein haufig auftretender und zu wenig beachteter Produktionsfehler, dessen Entstehung und Bekämpfung. Dt. Molkerei-Zeitung 103, 690-698.

### DEPOSITS and EXPERT SOLUTION

The applicant requests that a sample of the deposited micro-organisms stated below may only be made available to an expert, until the date on which the patent is granted.

The *Lactobacillus fermentum* strain CHCC12798 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32084.

The *Lactobacillus fermentum* strain CHCC12797 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32085.

The *Lactobacillus fermentum* strain CHCC14591 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32086.

The *Lactobacillus fermentum* strain CHCC14588 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32087.

The *Lactobacillus fermentum* strain CHCC15844 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32088.

The *Lactobacillus fermentum* strain CHCC15865 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32089.

The *Lactobacillus fermentum* strain CHCC15847 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32090.

The *Lactobacillus fermentum* strain CHCC15848 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32091.

The *Lactobacillus fermentum* strain CHCC15926 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-22 under the accession No.: 32096.

The *Lactobacillus fermentum* strain CHCC2008 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2009-05-19 under the accession No.: 22584.

The *Lactobacillus rhamnosus* strain CHCC15860 was deposited at German Collection of Microorganisms and Cell Cultures (Deutsche Sammlung von Mikroorganismen und Zellkulturen GmbH; DSMZ), Inhoffenstr. 7B, D-38124 Braunschweig deposited on 2015-07-16 under the accession No.: 32092.

The deposits were made according to the Budapest treaty on the international recognition of the deposit of microorganisms for the purposes of patent procedure.

## Claims

1. Bacterium of the species *Lactobacillus fermentum* selected from the group consisting of:
(a) the *Lactobacillus fermentum* strain deposited as DSM32086;
(b) the *Lactobacillus fermentum* strain deposited as DSM32087;
(c) the *Lactobacillus fermentum* strain deposited as DSM32089;
(d) the *Lactobacillus fermentum* strain deposited as DSM32091; or
(e) a mutant *Lactobacillus* strain obtainable from one the deposited bacteria according to (a) to (d), wherein the mutant is **characterized in that** it increases the pH of a fermented milk product comprising the mutant *Lactobacillus fermentum* during storage after fermentation in comparison to a milk product fermented with the same starter culture not containing the *Lactobacillus fermentum,*
wherein the increase in pH is at least by a value of 0.1, and
wherein the increase in pH is determined after storing a product fermented with a starter culture and the *Lactobacillus fermentum* in a concentration of at least 10⁷ cfu/g over 21 days at 25 °C.

2. The bacterium of the species *Lactobacillus fermentum* according to claim 1, wherein the fermented milk product comprising the mutant *Lactobacillus fermentum* maintains a pH above 4.0 when stored for at least 14 days at 25 °C, wherein the fermented milk product is obtained by a method comprising incubating a milk with the mutant *Lactobacillus fermentum* in a concentration of at least 10⁷ CFU/g and with a starter culture, fermenting until a pH of 4.6 is reached, shaking the fermented product and cooling.

3. The bacterium of the species *Lactobacillus fermentum* according to any one of claims 1 to 2, wherein the starter culture used for the preparation of the fermented milk product comprises LAB which are able to decrease the pH of a milk product during fermentation to a value of pH 4.6 in 10 hours or less.

4. The bacterium of the species *Lactobacillus fermentum* according to any one of claims 1 to 3, wherein the starter culture comprises a mixture of *Streptococcus thermophilus* and *Lactobacillus delbrueckii* subsp. *bulgaricus.*

5. Composition comprising at least one *Lactobacillus fermentum* strain according to claim 1 to 4.

6. The composition according to claim 5, wherein the *Lactobacillus fermentum* strain is in a concentration of at least 10⁷ CFU/g.

7. The composition according to claim 5 or 6, wherein the composition further comprises least one further bacterium selected from one or more of the following genera *Lactococcus spp., Bifidobacterium spp., Streptococcus spp., Lactobacillus spp., Leuconostoc spp., Pseudoleuconostoc spp., Pediococcus spp., Brevibacterium spp.* and *Enterococcus spp.*

8. The composition according to any one of claims 5 to 7, wherein the composition further comprises at least one further bacterium selected from *Lactobacillus delbrueckii subsp. bulgaricus, Streptococcus thermophilus, Lactobacillus lactis, Bifidobacterium animalis, Lactococcus lactis, Lactobacillus paracasei, Lactobacillus plantarum, Lactobacillus helveticus, Lactobacillus acidophilus,* and *Bifidobacterium breve.*

9. The composition according to any one of claims 5 to 8, wherein the composition further comprises at least one further bacterium selected from:
(a) *Lactobacillus rhamnosus* bacterium of strain CHCC15860 as deposited with the German Collection of Microorganisms and Cell Cultures (DSMZ) under accession No. DSM32092;
(b) *Lactobacillus rhamnosus* bacterium of strain CHCC5366 as deposited with the German Collection of Microorganisms and Cell Cultures (DSMZ) under accession No. DSM23035;
(c) *Lactobacillus rhamnosus* bacterium of strain CHCC12697 as deposited with the German Collection of Microorganisms and Cell Cultures (DSMZ) under accession No. DSM24616;
(d) *Lactobacillus paracasei* bacterium of strain CHCC12777 as deposited with the German Collection of Microorganisms and Cell Cultures (DSMZ) under accession No. DSM24651; and
(e) *Lactobacillus paracasei* bacterium of strain CHCC14676 as deposited with the German Collection of Microorganisms and Cell Cultures (DSMZ) under accession No. DSM25612.

10. The composition according to any one of claims 5 to 9, further comprising at least one cryoprotective compound.

11. The composition according to any one of claims 5 to 10, wherein the composition is a solid frozen or freeze dried starter culture comprising lactic acid bacteria in a concentration of at least 10⁹ colony forming units per g of frozen material or in a concentration of at least 10¹⁰ colony forming units per g of frozen material or in a concentration of at least 10¹¹ colony forming units per g of frozen material.

12. Use of the bacterium according to any one of claims 1 to 4 or the composition according to any one of claims 5 to 11 to produce a food, feed or pharmaceutical product.

13. Method of producing a fermented milk product comprising adding a *Lactobacillus fermentum* bacterium according to any one of claims 1 to 4 or the composition according to any one of claims 5 to 11 to milk or to a milk product and fermenting the mixture at a temperature between about 22°C and about 43°C until a pH of 4.6 or less than 4.6 is reached.

14. Food, feed or pharmaceutical product comprising the bacterium according any of claims 1-4 or the composition according to claims 5-11.

15. Food, feed or pharmaceutical product according to claim 14, obtainable by the method of claim 13.

## Patentansprüche

1. Bakterium der Spezies *Lactobacillus fermentum,* das aus der Gruppe ausgewählt ist, die aus Folgendem besteht:
(a) dem Stamm *Lactobacillus fermentum,* der als DSM32086 hinterlegt ist;
(b) dem Stamm *Lactobacillus fermentum,* der als DSM32087 hinterlegt ist;
(c) dem Stamm *Lactobacillus fermentum,* der als DSM32089 hinterlegt ist;
(d) dem Stamm *Lactobacillus fermentum,* der als DSM32091 hinterlegt ist; oder
(e) einem mutierten *Lactobacillus-Stamm,* der von einer der hinterlegten Bakterien gemäß (a) bis (d) erhaltbar ist, wobei der Mutant **dadurch gekennzeichnet ist, dass** er den pH-Wert eines fermentierten Milchproduktes, das das mutierte *Lactobacillus fermentum* umfasst, während der Lagerung nach der Fermentation im Vergleich zu einem Milchprodukt, das mit der gleichen Ausgangskultur fermentiert ist, die das *Lactobacillus fermentum* nicht enthält, erhöht,
wobei die Erhöhung des pH-Wertes zumindest um einen Wert von 0,1 ist, und
wobei die Erhöhung des pH-Wertes nach dem Lagern eines Produktes, das mit einer Ausgangskultur und dem *Lactobacillus fermentum* in einer Konzentration von zumindest 10⁷ cfu/g über 21 Tage bei 25 °C fermentiert wird, bestimmt wird.

2. Bakterium der Spezies *Lactobacillus fermentum* nach Anspruch 1, wobei das fermentierte Milchprodukt, das das mutierte *Lactobacillus fermentum* umfasst, einen pH-Wert über 4,0 beibehält, wenn es zumindest 14 Tage bei 25 °C gelagert wird, wobei das fermentierte Milchprodukt durch ein Verfahren erhalten wird, das Inkubieren einer Milch mit dem mutierten *Lactobacillus fermentum* in einer Konzentration von zumindest 10⁷ CFU/g und mit einer Ausgangskultur, Fermentieren, bis ein pH-Wert von 4,6 erreicht ist, Schütteln des fermentierten Produktes und Kühlen umfasst.

3. Bakterium der Spezies *Lactobacillus fermentum* nach einem der Ansprüche 1 bis 2, wobei die Ausgangskultur, die für die Herstellung des fermentierten Milchproduktes verwendet wird, Milchsäurebakterien (LAB) umfasst, die den pH-Wert eines Milchproduktes während der Fermentation in 10 Stunden oder weniger auf einen pH-Wert von 4,6 senken können.

4. Bakterium der Spezies *Lactobacillus fermentum* nach einem der Ansprüche 1 bis 3, wobei die Ausgangskultur ein Gemisch aus *Streptococcus thermophilus* und *Lactobacillus delbrueckii* susp. *bulgaricus* umfasst.

5. Zusammensetzung, umfassend zumindest einen *Lactobacillus fermentum-Stamm* nach Anspruch 1 bis 4.

6. Zusammensetzung nach Anspruch 5, wobei der *Lactobacillus fermentum-Stamm* in einer Konzentration von zumindest 10⁷ CFU/g ist.

7. Zusammensetzung nach Anspruch 5 oder 6, wobei die Zusammensetzung ferner zumindest ein weiteres Bakterium umfasst, das aus einer oder mehreren der folgenden Gattungen ausgewählt ist: *Lactococcus spp., Bifidobacterium spp., Streptococcus spp., Lactobacillus spp., Leuconostoc spp., Pseudoleuconostoc spp., Pediococcus spp., Brevibacterium spp.* und *Enterococcus spp.*

8. Zusammensetzung nach einem der Ansprüche 5 bis 7, wobei die Zusammensetzung ferner zumindest ein weiteres Bakterium umfasst, das aus *Lactobacillus delbrueckii subsp. bulgaricus, Streptococcus thermophilus, Lactobacillus lactis, Bifidobacterium animalis, Lactococcus lactis, Lactobacillus paracasei, Lactobacillus plantarum, Lactobacillus helveticus, Lactobacillus acidophilus* und *Bifidobacterium breve* ausgewählt ist.

9. Zusammensetzung nach einem der Ansprüche 5 bis 8, wobei die Zusammensetzung ferner zumindest ein weiteres Bakterium umfasst, das aus Folgendem ausgewählt ist:
(a) dem Bakterium *Lactobacillus rhamnosus* des Stammes CHCC15860 wie bei der Deutschen Sammlung von Mikroorganismen und Zellkulturen (DSMZ) unter der Zugangsnummer DSM32092 hinterlegt;
(b) dem Bakterium *Lactobacillus rhamnosus* des Stammes CHCC5366 wie bei der Deutschen Sammlung von Mikroorganismen und Zellkulturen (DSMZ) unter der Zugangsnummer DSM23035 hinterlegt;
(c) dem Bakterium *Lactobacillus rhamnosus* des Stammes CHCC12697 wie bei der Deutschen Sammlung von Mikroorganismen und Zellkulturen (DSMZ) unter der Zugangsnummer DSM24616 hinterlegt;
(d) dem Bakterium *Lactobacillus paracasei* des Stammes CHCC12777 wie bei der Deutschen Sammlung von Mikroorganismen und Zellkulturen (DSMZ) unter der Zugangsnummer DSM24651 hinterlegt; und
(e) dem Bakterium *Lactobacillus paracasei* des Stammes CHCC14676 wie bei der Deutschen Sammlung von Mikroorganismen und Zellkulturen (DSMZ) unter der Zugangsnummer DSM25612 hinterlegt.

10. Zusammensetzung nach einem der Ansprüche 5 bis 9, ferner umfassend zumindest eine kryoprotektive Verbindung.

11. Zusammensetzung nach einem der Ansprüche 5 bis 10, wobei die Zusammensetzung eine feste gefrorene oder gefriergetrocknete Ausgangskultur ist, die Milchsäurebakterien in einer Konzentration von zumindest 10⁹ koloniebildenden Einheiten pro g an gefrorenem Material oder in einer Konzentration von zumindest 10¹⁰ koloniebildenden Einheiten pro g an gefrorenem Material oder in einer Konzentration von zumindest 10¹¹ koloniebildenden Einheiten pro g an gefrorenem Material umfasst.

12. Verwendung des Bakteriums nach einem der Ansprüche 1 bis 4 oder der Zusammensetzung nach einem der Ansprüche 5 bis 11 zur Herstellung eines Lebensmittel-, Futter- oder pharmazeutischen Produktes.

13. Verfahren zum Herstellen eines fermentierten Milchproduktes, umfassend das Hinzufügen eines *Lactobacillus fermentum-*Bakteriums nach einem der Ansprüche 1 bis 4 oder der Zusammensetzung nach einem der Ansprüche 5 bis 11 zu Milch oder zu einem Milchprodukt und das Fermentieren des Gemischs bei einer Temperatur zwischen ungefähr 22 °C und ungefähr 43 °C, bis ein pH-Wert von 4,6 oder weniger als 4,6 erreicht ist.

14. Lebensmittel-, Futter- oder pharmazeutisches Produkt, das das Bakterium nach einem der Ansprüche 1-4 oder die Zusammensetzung nach Anspruch 5-11 umfasst.

15. Lebensmittel-, Futter- oder pharmazeutisches Produkt nach Anspruch 14, das durch das Verfahren nach Anspruch 13 erhaltbar ist.

## Revendications

1. Bactérie de l'espèce *Lactobacillus fermentum* choisie dans le groupe consistant en :
(a) la souche de *Lactobacillus fermentum* déposée sous DSM32086 ;
(b) la souche de *Lactobacillus fermentum* déposée sous DSM32087 ;
(c) la souche de *Lactobacillus fermentum* déposée sous DSM32089 ;
(d) la souche de *Lactobacillus fermentum* déposée sous DSM32091 ; ou
(e) une souche de *Lactobacillus* mutant pouvant être obtenue à partir de l'une des bactéries déposées selon (a) à (d), dans laquelle le mutant est **caractérisé en ce qu'**il augmente le pH d'un produit laitier fermenté comprenant le mutant *Lactobacillus fermentum* pendant un stockage après fermentation en comparaison à un produit laitier fermenté avec la même culture starter ne contenant pas le *Lactobacillus fermentum,*
dans laquelle l'augmentation de pH est d'au moins une valeur de 0,1, et
dans laquelle l'augmentation de pH est déterminée après stockage d'un produit fermenté avec une culture starter et le *Lactobacillus fermentum* à une concentration d'au moins 10⁷ cfu/g sur 21 jours à 25 °C.

2. Bactérie de l'espèce *Lactobacillus fermentum* selon la revendication 1, dans laquelle le produit laitier fermenté comprenant le mutant *Lactobacillus fermentum* maintient un pH au-dessus de 4,0 lorsqu'il est stocké pendant au moins 14 jours à 25 °C, dans laquelle le produit laitier fermenté est obtenu par une méthode comprenant l'incubation d'un lait avec le mutant *Lactobacillus fermentum* à une concentration d'au moins 10⁷ CFU/g et avec une culture starter, la fermentation jusqu'à ce qu'un pH de 4,6 soit atteint, le secouement du produit fermenté et le refroidissement.

3. Bactérie de l'espèce *Lactobacillus fermentum* selon l'une quelconque des revendications 1 à 2, dans laquelle la culture starter utilisée pour la préparation du produit laitier fermenté comprend LAB qui sont aptes à diminuer le pH d'un produit laitier pendant la fermentation à une valeur de pH 4,6 en 10 heures ou moins.

4. Bactérie de l'espèce *Lactobacillus fermentum* selon l'une quelconque des revendications 1 à 3, dans laquelle la culture starter comprend un mélange de *Streptococcus thermophilus* et *Lactobacillus delbrueckii* sous-espèce *bulgaricus.*

5. Composition comprenant au moins une souche de *Lactobacillus fermentum* selon la revendication 1 à 4.

6. Composition selon la revendication 5, dans laquelle la souche de *Lactobacillus fermentum* est à une concentration d'au moins 10⁷ CFU/g.

7. Composition selon la revendication 5 ou 6, dans laquelle la composition comprend en outre au moins une bactérie supplémentaire choisie parmi une ou plusieurs des genres suivants : *Lactococcus spp., Bifidobacterium spp*., *Streptococcus spp., Lactobacillus spp., Leuconostoc spp., Pseudoleuconostoc spp., Pediococcus spp., Brevibacterium spp.* et *Enterococcus spp.*

8. Composition selon l'une quelconque des revendications 5 à 7, dans laquelle la composition comprend en outre au moins une bactérie supplémentaire choisie parmi *Lactobacillus delbrueckii sous-espèce bulgaricus, Streptococcus thermophilus, Lactobacillus lactis, Bifidobacterium animalis, Lactococcus lactis, Lactobacillus paracasei, Lactobacillus plantarum, Lactobacillus helveticus*, *Lactobacillus acidophilus,* et *Bifidobacterium breve.*

9. Composition selon l'une quelconque des revendications 5 à 8, dans laquelle la composition comprend en outre au moins une bactérie supplémentaire choisie parmi :
(a) une bactérie *Lactobacillus rhamnosus* de la souche CHCC15860 telle que déposée auprès de la collection allemande de microorganismes et cultures cellulaires (DSMZ) sous le numéro d'accès DSM32092 ;
(b) une bactérie *Lactobacillus rhamnosus* de la souche CHCC5366 telle que déposée auprès de la collection allemande de microorganismes et cultures cellulaires (DSMZ) sous le numéro d'accès DSM23035 ;
(c) bactérie *Lactobacillus rhamnosus* de la souche CHCC12697 telle que déposée auprès de la collection allemande de microorganismes et cultures cellulaires (DSMZ) sous le numéro d'accès DSM24616 ;
(d) une bactérie *Lactobacillus paracasei* de la souche CHCC12777 telle que déposée auprès de la collection allemande de microorganismes et cultures cellulaires (DSMZ) sous le numéro d'accès DSM24651 ; et
(e) une bactérie *Lactobacillus paracasei* de la souche CHCC14676 telle que déposée auprès de la collection allemande de microorganismes et cultures cellulaires (DSMZ) sous le numéro d'accès DSM25612.

10. Composition selon l'une quelconque des revendications 5 à 9, comprenant en outre au moins un composé cryoprotecteur.

11. Composition selon l'une quelconque des revendications 5 à 10, dans laquelle la composition est une culture starter congelée ou cryodesséchée solide comprenant des bactéries d'acide lactique à une concentration d'au moins 10⁹ unités formant colonie par g de matière congelée ou à une concentration d'au moins 10¹⁰ unités formant colonie par g de matière congelée ou à une concentration d'au moins 10¹¹ unités formant colonie par g de matière congelée.

12. Utilisation de la bactérie selon l'une quelconque des revendications 1 à 4 ou de la composition selon l'une quelconque des revendications 5 à 11 pour produire un aliment, un produit alimentaire ou pharmaceutique.

13. Procédé de production d'un produit laitier fermenté comprenant l'addition d'une bactérie de *Lactobacillus fermentum* selon l'une quelconque des revendications 1 à 4 ou de la composition selon l'une quelconque des revendications 5 à 11 à du lait ou à un produit laitier et la fermentation du mélange à une température entre environ 22 °C et environ 43 °C jusqu'à ce qu'un pH de 4,6 ou de moins de 4,6 soit atteint.

14. Aliment, produit alimentaire ou pharmaceutique comprenant la bactérie selon l'une quelconque des revendications 1 à 4 ou la composition selon l'une quelconque des revendications 5 à 11.

15. Aliment, produit alimentaire ou pharmaceutique selon la revendication 14, pouvant être obtenu par le procédé de la revendication 13.
